# EUROPÄISCHE PATENTSCHRIFT

(11) **EP 0 533 700 B1**
(45) Veröffentlichungstag und Bekanntmachung des Hinweises auf die Patenterteilung: **13.04.1994**
(21) Anmeldenummer: 91910162.6
(22) Anmeldetag: 10.06.1991
(51) Int. Cl.: A61N 1/05

(54) **VORRICHTUNG ZUR EXTRAHIERUNG IN EINEM KÖRPERORGAN EINGEWACHSENER ELEKTRODENSPIRALEN**
DEVICE FOR EXTRACTING HELICAL ELECTRODES WHICH HAVE BECOME EMBEDDED IN BODY ORGANS
DISPOSITIF POUR L'EXTRACTION DE SPIRALES D'ELECTRODES INTRODUITES DANS UN ORGANE DU CORPS HUMAIN

(30) Priorität: 11.06.1990 DE 4018681
(43) Veröffentlichungstag der Anmeldung: 31.03.1993
(73) Patentinhaber: VascoMed Institut für Kathetertechnologie GmbH, D-79553 Weil am Rhein (DE)
(72) Erfinder: HÖCHERL, Manfred, D-7858 Efringen-Kirchen (DE); REINHARDT, Jörg, D-7889 Grenzach-Wyhlen (DE)
(74) Vertreter: Rackette, Karl, Dipl.-Phys. Dr.-Ing
(86) Internationale Anmeldenummer: DE9100490
(87) Internationale Veröffentlichungsnummer: WO9119532

(56) Entgegenhaltungen:
- US-A- 4 471 777
- US-A- 4 574 800
- Research Disclosure, Nr.299, März 1989 (New York US) J.E.Graf et al: "Coil traction stylet"

## Beschreibung

Die Erfindung betrifft eine Vorrichtung zur Extrahierung einer im Herzen eingewachsenen Herzschrittmacherelektrodenspirale durch Ausübung einer Zugkraft auf diese mit einem in das Innenlumen der Elektrodenspirale einführbaren Zugelement mit einem aufweitbaren oder aufspreizbaren Zugelementkopf, wobei das Zugelement aus einem Zugelementmantel mit daran befestigtem Zugelementkopf und einem in diesem längsverschiebbaren Innendraht besteht.

Eine häufig angewandte Methode, um nichtfunktionierende oder infizierte Herzschrittmacherelektroden aus dem Körper zu entfernen ist die sogenannte Dauerzugbelastung, bei der die Elektrode in der Herzschrittmachertasche freigelegt wird und mit einer Dauerzugbelastung zwischen 100 bis 500 Gramm über Stunden und Tage beaufschlagt wird, bis sich die Elektrode aus Gewebeverwachsungen löst. Ein Gewicht wirkt mittels einer Seilführung als Zugbelastung auf die Elektrode.

Nachteilig bei dieser Methode ist, daß die Dauerzugbelastung oft über mehrere Tage angewandt werden muß, wobei der Patient an der Zugvorrichtung und im Bett verweilen muß.

Es ist ferner bekannt, zum Extrahieren nicht funktionierender oder infizierter, eingewachsener Herzschrittmacherelektroden einen Schlingenkatheter zu verwenden, wobei eine Schlinge über die Herzkatheterelektrode bis zum Einwachsungsort vorgeschoben und fixiert wird. Eine Zugkraft kann dann direkt vor Ort angewendet werden.

Von Nachteil bei diesen bekannten und überwiegend angewandten Verfahrensweisen erscheint, daß ein Schlingenkatheter nicht bei infizierten Herzschrittmachertaschen oder Herzkatheterelektroden angewendet werden soll, da es beim Vorschieben des Schlingenkatheters über die Elektrode zu einer Keimverschleppung ins Herz kommen kann. Bei Anwendung der Dauerzugbelastung wird der Patient durch die erzwungene langdauernde Ruhehaltung im Bett psychisch und physisch in seinem Befinden schwer beeinträchtigt.

Aus der US 4 574 800 ist eine Vorrichtung gemäß dem Oberbegriff des Anspruch 1 bekannt, bei der an dem Zugdraht eine Kugel befestigt ist, die, wenn sie zurückgezogen wird, den Zugelementmantel aufspreizt und so diesen mit der Elektrodenspirale verklemmt. Dann kann die gesamte Einheit mit der Elektrode zurückgezogen werden, Diese Vorrichtung weist den Nachteil auf, daß nach der erreichten Verklemmung eine Relativbewegung zwischen Zugelementmantel und Zugdraht ausgeschlossen werden muß, da sich ansonsten der Zugriff der Kugel lösen könnte. Dies führt zu einer schwer manipulierbaren Auszugseinrichtung mit entgegengesetzt zu bewegenden und haltenden Elementen.

Wird dagegen die Kugel derart verklemmt, daß sie unverrückbar sitzt, und damit in einfacherer Weise an dem Zugdraht und dem Zugmantel gezogen werden kann, entfällt die Möglichkeit, den Auszugsvorgang abzubrechen, da über die beiden Elemente Zugdraht und Zugmantel keine entsprechende Druckkraft mehr ausübbar ist, um die Kugel zu befreien. Dies ist um so mehr der Fall bei Benützung der alternativen Zugelementköpfe mit zylinddrischem Durchmesser.

Aus der Research Disclosure, Nr. 299, März 1989, Seite 223 ist ein Extrahierungsinstrument bekannt, das über rückwärtig gerichtete Widerhaken verfügt, die durch abgeschrägte Ausnehmungen aus einem hypodermischen Schlauch gebildet worden sind. Diese Vorrichtung weist den Nachteil auf, daß sie in der Praxis nur schwer einsetzbar ist, da für ein effektives Greifen der Widerhaken der Durchmesser des Schlauches etwas größer als der Innendurchmesser des Lumens der Elektrodenspirale sein muß, so daß das Extrahierungsinstrument nur mit erheblichem, kaum aufzuwendenen Druck in die Elektrodenspirale einführbar ist.

Der Erfindung liegt das Ziel zugrunde, das Extrahieren von bereits implantierten, im Herzen eingewachsener, nichtfunktionsfähiger oder infizierter Elektrodenspiralen zu erleichtern. Die dazu erforderliche Zugkraft soll direkt am Einwachsungsort ausgeübt werden, und eine Infektion des Herzens z. B. durch Keimverschleppung, wie sie z. B. durch das Vorschieben der Extraktionsvorrichtung hervorgerufen werden kann, soll ausgeschlossen sein.

Ausgehend von dem oben genannten Stand der Technik liegt der Erfindung die Aufgabe zugrunde ein einfach zu benutzendes Extrahierungsinstrument anzugeben, daß in der Einführungsphase, der Auszugsphase und beim Abbruch der Behandlung durch einen Operateur leicht zu handhaben ist und für den Patienten eine größtmögliche Sicherheit beim Extrahieren bietet.

Zur erfinderischen Lösung dieser Aufgabe wird ein mit den aus dem zitierten Oberbegriff bekannten Merkmalen versehenes Extrahierungsinstrument vorgeschlagen, das dadurch gekennzeichnet ist, daß der Zugelementkopf über wenigstens zwei an einem mit dem Zugelement verbundenen Schirmkopf angebrachten, ausspreizbaren Widerhaken verfügt, die sich nach Relativverschiebung zwischen Zugelement und Zugelementkopf bei Bewegungsrichtungsumkehr des Zugelements in die Wandung des Innenlumens verhaken.

Die Benutzung dieser Vorrichtung nach der Erfindung bedeutet für den Arzt bzw. Chirurgen eine erhebliche Erleichterung und für den Patienten Schonung in physischer und psychischer Hinsicht. Die Extraktion eingewachsener Elektroden wird nach der erfindungsgemäßen Lehre auch mit größerer Sicherheit und Einfachheit durchgeführt, weil sowohl ungezielte Manipulationen von Hand vermieden werden und auch der apparative Aufwand weitgehend entfällt.

Dadurch, daß das Zugelement aus einem Zugelementmantel mit daran befestigten Zugelementkopf und einem in diesem längsverschiebbaren Innendraht besteht wird eine hervorragende Manipulierbarkeit der Vorrichtung ermöglicht, weil der Innendraht die Ausführung von bestimmten Ein- und Verstellfunktionen gestattet.

Dadurch, daß der Zugelementkopf mit wenigstens zwei mittels Relativverschiebung des Innendrahtes ausstellbaren, an einem Schirmkopf angeordneten Widerhaken versehen ist, kann ein und dasselbe Extrahierungsinstrument bei einer Vielzahl von gleichartigen, aber unterschiedlich großen Elektrodenspiralen eingesetzt werden, womit sich die Sicherheit und die Erfolgsrate des Eingriffs erhöht.

Eine besonders einfache Einführung des Instruments ist bei einer besonders vorteilhaften Variante gegeben, die in der Weise ausgebildet ist, daß der Zugelementkopf eine Kammer zur Aufnahme der wenigstens zwei an dem mit dem Zugelement verbundenen Schirmkopf angebrachten und ausspreizbaren Widerhaken enthält, die nach Relativverschiebung zwischen Zugelement und Zugelementkopf durch Herausdrücken aus der Kammer freigebbar sind.

Schließlich ist es nach einem zusätzlichen Vorschlag möglich, diese letztere Ausbildungsform dadurch zu vervollkommnen, daß der Schirmkopf mit dem Ende des Zugelements lösbar angeordnet ist.

Diese verschiedenen Ausbildungsformen, welche noch weitere Varianten und auch gewisse Verfeinerungen ermöglichen, beruhen auf einem der Grundgedanken der Erfindung, durch Einführung des Zugelements mit einem Zugelementkopf in das Innenlumen der Elektrode, diesen Zugelementkopf in der Weise auszubilden, daß er sich form- oder kraftschlüssig an die Innenwandung der Elektrodenspirale anlegt oder verklemmt bzw. ankrallt oder verhakt, wodurch eine zumindest soweit zugfeste Verbindung geschaffen wird, um dadurch eine individuell bemeßbare und ausübbare Zugkraft - von Hand - zum Ansatz zu bringen, und zwar diesen Ansatz in unmittelbarer Nähe der Einwachsstelle im Körper angreifen zu lassen.

Durch den Innendraht in dem Zugelement ist es vor allem möglich, die verschiedenartig gestaltbaren Elemente zu aktivieren und damit zur Verbindung mit der Innenwandung des Elektrodenlumens zu bringen, um die Zugkraft an den gewünschten Stellen auf die Elektrode zu übertragen.

Wie bereits erwähnt, kann in Einzelfällen das Extrahieren der zu stark eingewachsenen Elektrodenspirale auf die beschriebene Weise mißlingen, so daß das Zugelement entfernt werden soll. Dies gelingt nach der weiteren Erfindung durch Herausdrehen aus den Spiralwindungen der Elektrode, und zu diesem Zweck ist vorgesehen, daß an das bedienerseitige Ende des Zugelements bzw. des Zugelementmantels eine handbetätigbare Vorrichtung zur Ausübung und Übertragung eines Drehmoments anschließbar ist, und in Abwandlung hiervon kann die Vorrichtung in der Weise ausgebildet sein, daß im Bereich der bedienerseitigen Enden des Zugelements und des Innendrahtes Vorrichtungen zum Herstellen einer drehfesten Verbindung zwischen Zugelement und Innendraht angeordnet sind. Zusätzlich besteht die Möglichkeit, daß mit dem bedienerseitigen Ende des Zugelements und/oder des Innendrahtes ein Motor zur Übertragung eines Drehmoments anschließbar ist. Zur Synchronisierung der Drehbewegung zwischen dem Zugelement und dem Innendraht kann ferner vorgesehen sein, daß zwischen dem Griff und dem Haltering eine drehfeste Verbindung herstellbar ist. Weiterhin kann es zweckmäßig sein, wenn der Griff mit einer Ankuppelvorrichtung für den mit einer Kuppelmöglichkeit versehenen Motor versehen ist.

Zur Bedienung der Vorrichtung sind ein Griff und ein Haltering vorgesehen, welche mit dem Innendraht und dem Zugelementmantel verbunden sind.

Weitere Merkmale und Einzelheiten der Erfindung sind anhand der in der Zeichnung dargestellten Ausführungsbeispiele näher erläutert.

Es zeigen:
- Fig. 1: die Vorrichtung nach der Erfindung in der einfachsten Ausführung im Schnitt beim Einführen in die Elektrodenspirale,
- Fig. 2: die Vorrichtung nach Fig. 1 verhakt in die Elektrodenspirale,
- Fig. 3: die Vorrichtung in einer weiteren Ausführung beim Einführen in die Elektrodenspirale,
- Fig. 4: die Vorrichtung nach Fig. 3 im Schnitt, verhakt in die Elektrodenspirale,
- Fig. 5: die Vorrichtung in eine Abwandlung der Ausführung nach Fig. 4 im Schnitt in der Phase des Einführens des Zugelements, wenn die Spreiznasen oder Widerhaken noch eingefahren sind,
- Fig. 6: die Vorrichtung gemäß Fig. 5 mit ausgefahrenen Spreiznasen,
- Fig. 7a/b: eine vereinfachte Darstellung der Ausbildung für einen Motorantrieb zum Herausdrehen,
- Fig. 8: einen Längsschnitt gemäß Fig. 7a.

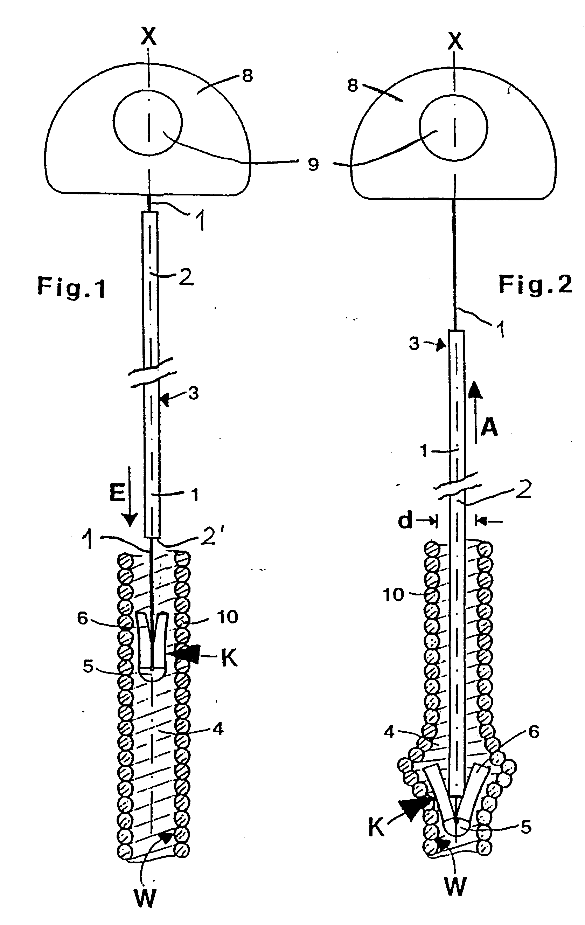

Die in den Fig. 1 und 2 dargestellte Ausführung der erfindungsgemäßen Vorrichtung zeigt im Schnitt in schematischer Darstellung die Elektrodenspirale 10 und das Zugelement 3, welches in das Innenlumen 4 eingeführt wird.

Am Ende des Zugelements 3 ist ein Zugelementkopf K angebracht, an welchem wenigstens zwei, vorzugsweise aber drei oder vier Widerhaken 6 befestigt sind. Die Anordnung und Ausbildung ist so getroffen, daß diese Widerhaken 6 unter der Wirkung einer permanent wirkenden Kraft das Bestreben haben, sich nach außen aufzuspreizen. Vorzugsweise sind der Zugelementkopf K und die Widerhaken 6 aus einem Kunststoff hergestellt und einstückig gearbeitet. Dabei kann die Formgestaltung derart ausgelegt sein, daß die Widerhaken unter einer permanent wirkenden Spreizspannung stehen. Entgegen dieser Spannung legen sie sich beim Einführen in die Elektrodenspirale an deren Wandung W an. Wird nun nach Erreichen der Endlage des Zugelementkopfes K in der Nähe des Einwachsungsorts der Elektrodenspirale durch Zug entgegen der Einführrichtung E in Richtung A bewegt, dann haken sich die Widerhaken 6 an der Wandung W des Innenlumens 4 der Elektrodenspirale ein und verkeilen sich, so daß eine Zugkraft auf die Elektrode ausgeübt werden kann. Zur Handhabung ist das Zugelement 3 mit einem Griff 8 mit Griffloch 9 versehen.

Die Ausbildungsweise kann auch so getroffen sein, daß der Zugelementkopf K mit seinem Schirmkopf 5 nicht unlösbar mit dem Zugelement 3 verbunden ist, sondern daß die Verbindung nur kraftschlüssig ist und sich bei Überschreiten einer vorgesehenen Grenzzugkraft löst; diese Grenzzugkraft kann derart bemessen sein, daß Verletzungen an der Einwachsungsstelle des Körpers des Patienten vermieden werden.

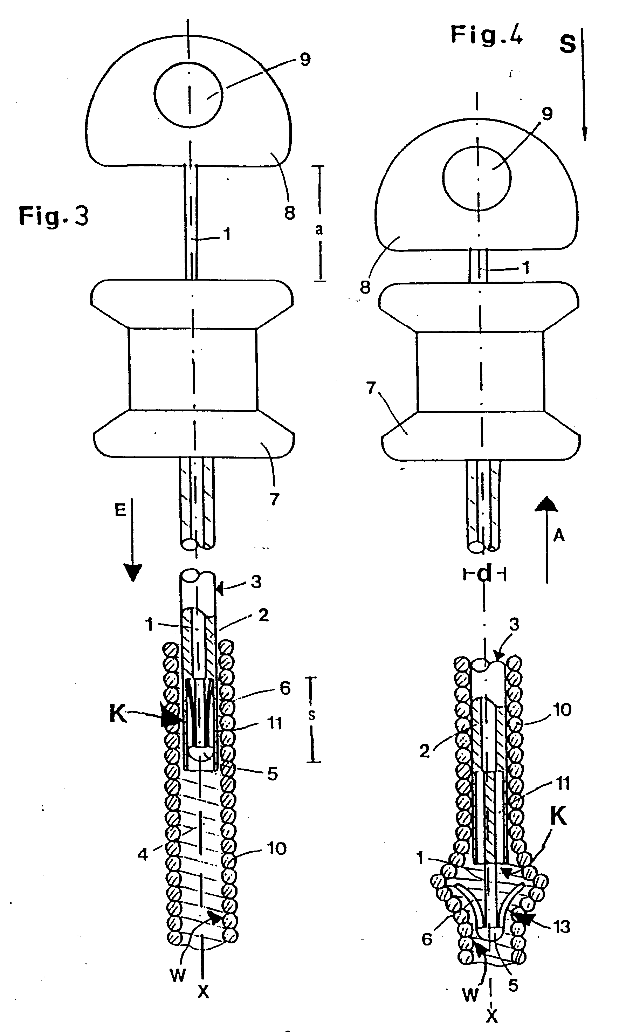

Die folgenden Ausführungen beziehen sich auf die Ausbildungsformen nach den Fig. 3 bis 6.

Die Vorrichtung wird mit dem Zugelement 3 in Einführrichtung E in das Innenlumen 4 einer Elektrodenspirale 10 eingeführt, die beispielsweise eine im Bereich der Herzschrittmachertasche freigelegte Herzkatheterelektrode ist.

Das aus Fig. 3 bis 6 ersichtliche Zugelement 3 besteht aus einem im Zugelementmantel 2 längsverschiebbaren Innendraht 1. Der am Zugelementmantel 2 befestigte Zugelementkopf K wird bis in den Bereich des Einwachsungsortes im Herz verschoben.

In der abgewandelter Ausführung nach den Fig. 3 und 4 ist am Ende des Zugelementmantels 2 eine Schirmkammer 11 angebracht, und am Ende des Innendrahtes 1 befindet sich ein Schirmkopf 5 mit an diesem befestigten schirmartigen Widerhaken 6, wie aus Fig. 3 ersichtlich. Am anderen Ende des Zugelementmantels 2 ist der Haltering 7 angebracht, während der Innendraht 1 mit dem Griff 8 verbunden ist. Durch Zueinanderbewegen von Haltering 7 und Griff 8 wird der Innendraht 1 gegenüber dem Zugelementmantel 2 verschoben und der Schirmkopf 5 mit den daran befestigten Widerhaken 6 gleitet aus der Schirmkammer 1 heraus, wobei sich die Widerhaken 6 schirmartig entfalten. Durch eine Bewegungsumkehr zwischen Innendraht 1 und Zugelementmantel 2 werden nun die an der Wandung W des Innenlumens 4 anliegenden Widerhaken 6 Widerstand finden und sich einhaken, so daß dadurch eine zugfeste Verbindung zwischen dem Zugelement 3 und der Elektrode 10 geschaffen und es ermöglicht wird, die letztere von Hand herauszuziehen.

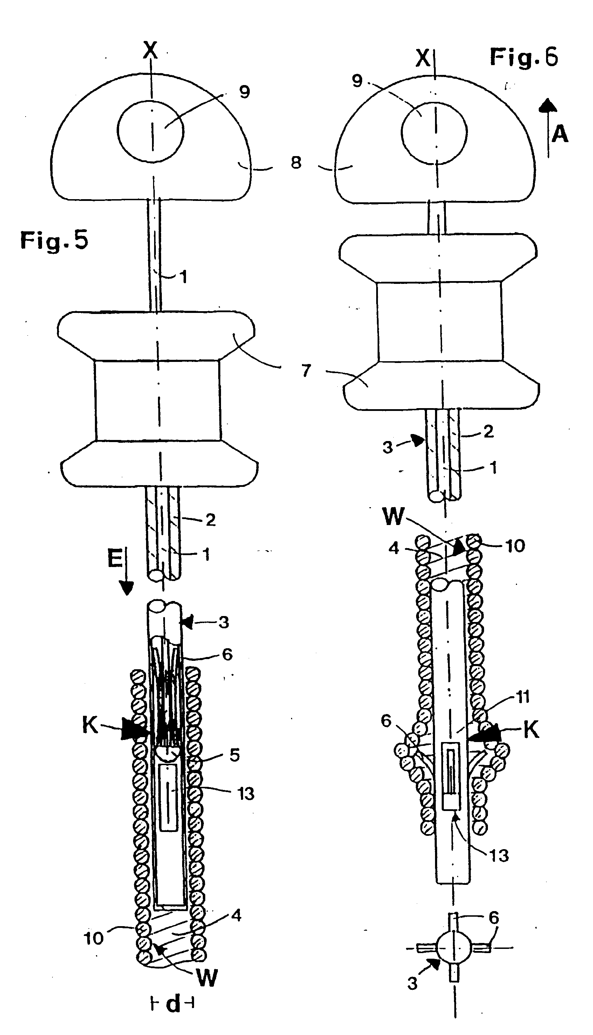

Eine weitere Abwandlung ist in den Fig. 5 und 6 dargestellt. Dort ist die Schirmkammer 11 völlig geschlossen, jedoch mit den jedem Widerhaken 6 bzw. jeder Spreiznase 16 zugeordneten Durchlaßöffnungen 13 versehen, die derart angeordnet und gestaltet sind, daß nach Erreichen der Endposition des Schirmkopfes 5 in seiner am weitesten vorgeschobenen Lage in der Schirmkammer 11 und anschließender Bewegungsumkehr des Innendrahtes 1 gegenüber dem Zugelementmantel 2 die Widerhaken 6 (bzw. Spreiznasen 16) durch die Durchlaßöffnungen 13 hindurchtreten und auf diese Weise nach außen gespreizt und zur Anlage an der Wand W des Innenlumens 4 gebracht werden. Darauf erfolgt, ebenso wie in den Fig. 3 und 4 gezeigt, infolge des Andrückens an die Wand W deren Verformung und die kraft- oder formschlüssige Verbindung mit der Elektrodenspirale 10.

Dabei kann nach einem wesentlichen Vorteil durch eine Relativverschiebung von Innendraht 1 gegen den Zugelementmantel 2 entgegen der Zugrichtung, also in Richtung E (Fig. 5), ein Zurückziehen der ausgespreizten Widerhaken 6 bzw. Spreiznasen 16 bewirkt und die Vorrichtung durch Zug am Haltering 7 herausgezogen bzw. entfernt werden. Außerdem besteht die weitere Möglichkeit, die Verbindung zwischen dem Innendraht 1 und dem Schirmkopf 5 derart auszubilden, daß sie sich bei Überschreiten eines Grenzwertes an Zugkraft löst.

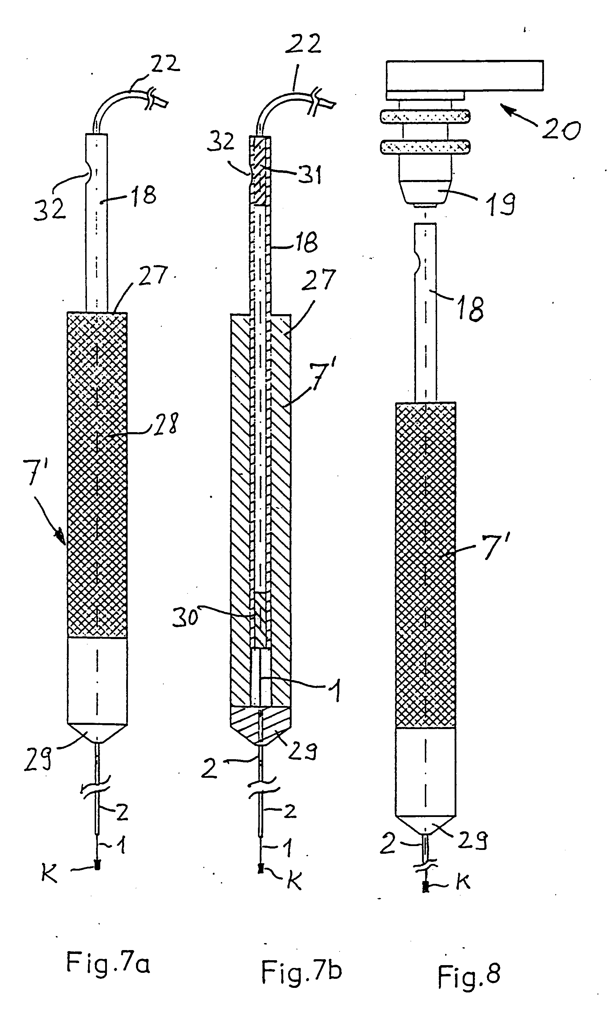

Wie aus den Fig. 7a, 7b und 8 ersichtlich ist, kann der Griff 8 mit einer Vorrichtung 21 - Halteknopf und Nut - versehen sein, mittels welcher eine drehfeste Verbindung zwischen dem Innendraht 1 - Haltering 7 - und dem Zugelement 3 - Griff 8 - herausstellbar ist. Mittels einer Ankuppelvorrichtung 18 und einer entsprechenden Kupplung 19 kann der Motor 20 mit dem Griff 8 derart verbunden werden, daß das Drehmoment auf Zugelement 3 und Innendraht 1 übertragen wird.

## Patentansprüche

1. Vorrichtung zur Extrahierung einer im Herzen eingewachsenen Herzschrittmacherelektrodenspirale durch Ausübung einer Zugkraft auf diese mit einem in das Innenlumen (4) der Elektrodenspirale (10) einführbaren Zugelement (3) mit einem aufweitbaren oder aufspreizbaren Zugelementkopf (K), wobei das Zugelement (3) aus einem Zugelementmantel (2) mit daran befestigtem Zugelementkopf (K) und einem in diesem längsverschiebbaren Innendraht (1) besteht, **dadurch gekenn****zeichnet**, daß der Zugelementkopf (K) über wenigstens zwei an einem mit dem Zugelement (1) verbundenen Schirmkopf (5) angebrachten, ausspreizbaren Widerhaken (6) verfügt, die sich nach Relativverschiebung zwischen Zugelement (1) und Zugelementkopf (K) bei Bewegungsrichtungsumkehr des Zugelements (3) in die Wandung (W) des Innenlumens (4) verhaken.

2. Vorrichtung nach Anspruch 1, dadurch gekennzeichnet, daß der Zugelementkopf (K) eine Kammer (11) zur Aufnahme der wenigstens zwei Widerhaken (6) enthält, die nach Relativverschiebung zwischen Zugelement (1) und Zugelementkopf (K) durch Herausdrücken aus der Kammer (11) freigebbar sind.

3. Vorrichtung nach Anspruch 1 oder Anspruch 2, dadurch gekennzeichnet, daß die Kammer (11) in Spiralachsrichtung (X) gerichtete Durchlaßöffnungen (13) für die am Schirmkopf (5) angebrachten Spreiznasen (16) oder Widerhaken (6) aufweist, durch welche letztere bei Relativverschiebung zwischen Zugelement (1) und Zugelementkopf (K) herausdrück- und spreizbar und wieder einziehbar sind.

4. Vorrichtung nach einem der Ansprüche 1 bis 3, dadurch gekennzeichnet, daß der Schirmkopf (5) am Ende des Zugelements (3) von diesem lösbar angeordnet ist.

5. Vorrichtung nach einem der Ansprüche 1 bis 4, dadurch gekennzeichnet, daß der Innendraht (1) an einem Griff (8) und der Zugelementmantel (2) an einem Haltering (7) angeschlossen ist.

6. Vorrichtung nach Anspruch 5, dadurch gekennzeichnet, daß der Griff (8) ein Griffloch (9) aufweist.

7. Vorrichtung nach einem der Ansprüche 1 bis 6, dadurch gekennzeichnet, daß an das bedienerseitige Ende des Zugelements bzw. des Zugelementmantels (2) eine handbetätigbare Vorrichtung zur Ausübung und Übertragung eines Drehmoments anschließbar ist.

8. Vorrichtung nach einem oder mehreren der Ansprüche 1 bis 7, dadurch gekennzeichnet, daß im Bereich der bedienerseitigen Enden des Zugelements (3) und des Innendrahtes (1) Vorrichtungen (21) zum Herstellen einer drehfesten Verbindung zwischen Zugelement (3) und Innendraht (4) angeordnet sind.

9. Vorrichtung nach wenigstens einem der Ansprüche 6 bis 8, dadurch gekennzeichnet, daß mit dem bedienerseitigen Ende des Zugelements (3) oder/und des Innendrahtes (1) ein Motor (20) zur Übertragung eines Drehmoments anschließbar ist.

10. Vorrichtung nach Anspruch 5 und 7, dadurch gekennzeichnet, daß zwischen dem Griff (8) und dem Haltering eine drehfeste Verbindung herstellbar ist.

11. Vorrichtung nach Anspruch 8, dadurch gekennzeichnet, daß der Griff (8) mit einer Ankuppelvorrichtung (18) für den mit einer Kuppelmöglichkeit (19) versehenen Motor (20) versehen ist.

## Claims

1. Device for extracting a cardiac pacemaker electrode coil, which is embedded in the heart, by exerting a tensile force on this coil using a pulling element (3) which can be introduced into the inner lumen (4) of the electrode coil (10) and has a pulling element head (K) which can be widened or spread open, the pulling element (3) consisting of a pulling element casing (2), with pulling element head (K) secured thereon, and of an inner wire (1) which is longitudinally displaceable in this casing, characterized in that the pulling element head (K) has at least two barbs (6) which can be spread open and are arranged on a protective cap (5) connected to the pulling element (1), these barbs (6) being hooked into the wall (W) of the inner lumen (4) after relative displacement between pulling element (1) and pulling element head (K) upon the reversal of the direction of movement of the pulling element (3).

2. Device according to Claim 1, characterized in that the pulling element head (K) comprises a chamber (11) for receiving the at least two barbs (6) which can be released by being pressed out from the chamber (11) after relative displacement between pulling element (1) and pulling element head (K).

3. Device according to Claim 1 or Claim 2, characterized in that the chamber (11) has, directed in the axial direction (X) of the coil, openings (13) through which the spreading lugs (16) or barbs (6) arranged on the protective cap (5) can be pressed out and spread and once more drawn in upon relative displacement between pulling element (1) and pulling element head (K).

4. Device according to one of Claims 1 to 3, characterized in that the protective cap (5) is arranged at the end of the pulling element (3) in such a way that it can be detached therefrom.

5. Device according to one of Claims 1 to 4, characterized in that the inner wire (1) is attached to a handle (8) and the pulling element casing (2) is attached to a holding ring (7).

6. Device according to Claim 5, characterized in that the handle (8) has a handle hole (9).

7. Device according to one of Claims 1 to 6, characterized in that a manually activatable device for exerting and transmitting a torque can be connected to the operator end of the pulling element or pulling element casing (2).

8. Device according to one or more of Claims 1 to 7, characterized in that devices (21) for producing a rotationally fixed connection between pulling element (3) and inner wire (4) are arranged in the area of the operator ends of the pulling element (3) and of the inner wire (1).

9. Device according to at least one of Claims 6 to 8, characterized in that a motor (20) for transmitting a torque can be connected to the operator end of the pulling element (3) and/or the inner wire (1).

10. Device according to Claim 5 and 7, characterized in that a rotationally fixed connection can be produced between the handle (8) and the holding ring.

11. Device according to Claim 8, characterized in that the handle (8) is provided with a coupling device (18) for the motor (20) which is provided with a coupling possibility (19).

## Revendications

1. Dispositif pour l'extraction d'électrodes de stimulation cardiaque introduites dans le coeur, apte à exercer une force de traction sur lesdites électrodes au moyen d'un élément de traction (3) introduit dans la cavité interne (4) d'une électrode en spirale (10) et équipé d'une tête de traction (K) capable de s'écarter ou de s'élargir, l'élément de traction (3) comportant un corps de traction (2) auquel est fixé la tête de traction (K) et dans lequel coulisse longitudinalement un fil intérieur (1), caractérisé en ce que la tête de traction (K) comporte au moins deux crochets (6) rapportés et écartables reliés à l'élément de traction (1) par une tête en pointe de parapluie (5), qui après un mouvement relatif entre l'élément de traction (1) et la tête de traction (K) s'accrochent dans la paroi (W) de la cavité interne (4) par marche arrière de l'élément de traction (3).

2. Dispositif selon la revendication 1, caractérisé en ce que la tête de traction (K) comporte une chambre (11) pour la prise des crochets (6) qui sont libérables de la chambre par une pression de sortie exercée par le mouvement relatif entre l'élément de traction (1) et la tête de traction (K).

3. Dispositif selon l'une des revendications 1 à 2 caractérisé en ce que la chambre (11) présente des fentes (13) dirigées selon l'axe (X) de la spirale pour le passage des crochets (6) ou des becs (16) accrochés à la tête en pointe de parapluie (5), lesquels peuvent sortir et s'écarter et à nouveau rentrer par les fentes suite au mouvement relatif entre élément de traction (1) et tête de traction (K).

4. Dispositif selon l'une des revendications 1 à 3, caractérisé en ce que la tête en pointe de parapluie (5) est placée à l'extrémité de l'élément de traction (3) sans y être attachée.

5. Dispositif selon l'une des revendications 1 à 4 caractérisé en ce que le fil intérieur (1) est fixé à une poignée (8), et le corps de traction (2) est fixé à un anneau support (7).

6. Dispositif selon la revendication 5 caractérisé en ce que la poignée (8) présente un trou de préhension (9).

7. Dispositif selon l'une des revendications 1 à 6, caractérisé en ce qu'un dispositif manuel pour exercer et transmettre un mouvement rotatif est fixé à l'extrémité active de l'élément de traction tel que le corps de traction (2).

8. Dispositif selon l'une au moins des revendications 1 à 7, caractérisé en ce que des moyens (21) pour réaliser une liaison tournante entre l'élément de traction (3) et la cavité interne (4) sont prévus à proximité de l'extrémité active de l'élément de traction (3) et du fil intérieur (1).

9. Dispositif selon l'une au moins des revendications 6 à 8 caractérisé en ce qu'un moteur (20) est fixé à l'extrémité active de l'élément de traction (3) et/ou du fil interne (1) pour exercer un mouvement rotatif.

10. Dispositif selon les revendications 5 et 7 carcatérisé en ce que entre la poignée (8) et l'anneau support est prévue une liaison rotative.

11. Dispositif selon la revendication 8 caractérisé en ce que la poignée (8) est équipée d'un dispositif d'accouplement (18) avec la tête d'accouplement (19) d'un moteur (20).
